# EUROPEAN PATENT APPLICATION

(11) **EP 3 064 156 A1**
(43) Date of publication of application: **07.09.2016**
(21) Application number: 15157597.4
(22) Date of filing: 04.03.2015
(51) Int. Cl.: A61B 17/16

(54) **DRILL MEMBER AND DRILL KIT**

(71) Applicant: Oticon Medical A/S, 2765 Smørum (DK)
(72) Inventor: Eriksson, Thomas, DK-2765 Smørum (DK); Johansson, Martin, DK-2765 Smørum (DK)
(74) Representative: Hauge, Christian

(57) **Abstract**

According to an embodiment, a drill member for making a blind hole in a bone under a soft tissue for installing an implant is disclosed. The drill member includes a stepped drill bit that includes a conical section, an intermediate section and a proximal section. The drill bit includes a conical width of a conical profile including an extrapolated outer surface of the conical section, an intermediate width of an intermediate profile comprising an extrapolated outer surface of the intermediate section and a proximal width of a proximal profile comprising an extrapolated outer surface of the proximal section. The conical width and/ or intermediate width is smaller than the proximal width. The proximal profile comprising the proximal width is adapted to receive at least a part of a first section of a drill head of a second drill member.

## Description

### FIELD

The disclosure generally relates to a device and a method for installing an implant. The disclosure more particularly relates to a device and method for installing an implant such as a bone-anchored hearing aid, screws and abutments for cranio-facial prostheses and maxillofacial retention systems.

### BACKGROUND

Medical implants such as a bone anchored hearing aids are applied for the rehabilitation of patients suffering from hearing losses for which traditional hearing aids are insufficient. A typical bone anchored hearing aid consists of an external hearing aid provided with a vibrating transducer connected to a skin-penetrating abutment through a coupling. The abutment may have an interconnection to a screw-shaped fixture anchored in the skull bone. The fixture is typically made of titanium and may be provided with a flange to prevent the fixture from being pushed through the skull bone when exposed to a sudden accidental impact.

The abutment penetrates the skin and the subcutaneous tissue in order to establish a direct coupling (direct bone conduction) from a hearing aid processor to the skull bone.

The methods for installing bone anchored hearing aid implant systems are moving toward minimally invasive methods that can be performed quickly in order to minimize intra-and post-operative problems, to achieve a predictable outcome, and achieve better cosmetic results.

However, the existing incision techniques are rather complicated and requires that a flap area to be provided by making an incision. Typically, a scalpel is used to make an incision down to the periosteum along a marking of the incision area and to separate the tissue from the underlying periosteum. Further, all subcutaneous tissue in the graft area is separated from the periosteum. Besides the subcutaneous tissue needs to be carefully separated from the skin graft, and all hair follicles needs to be removed. Furthermore, some level of manual skin thinning typically is required to be performed.

Some attempts have been made to avoid the linear incision techniques to install implants for bone anchored hearing aids. Some of these attempts include punch techniques. The techniques apply a standard biopsy punch that is used to provide a circular incision of 5-12 mm.

These techniques are associated with a number of drawbacks. The drill interfaces the soft tissue and hereby introduces the risk of damaging the tissue due to friction, heat and tearing caused by the action of the drill.

These punching techniques apply punching holes larger than 5 mm in order to allow for introducing irrigation fluid (to cool the bone tissue) during the drilling process and also for providing sufficient visibility. These large punch diameters are not optimal for the soft tissue abutment interface. A large circular incision will prolong the healing time and introduce the risk of granulation tissue formation and subsequent infection. Moreover, the skin thickness needs to be determined pre and/or intra operatively.

Thus there is need for a method and device that at least reduces or even eliminates these drawbacks of the prior art.

### SUMMARY

According to an embodiment, the disclosure provides a procedure facilitating an increased control in making a blind hole for installing an implant and a more predictable outcome compared to the incision techniques used today. The procedure includes a less invasive method that requires a shorter healing time when compared to prior art techniques. The method is relatively faster, uncomplicated as well as patient and economy friendly. According to another embodiment, the disclosed method that facilitates irrigation and/ or correct drill depth. Furthermore, the disclosure provides devices that facilitate increasing certainty of making a desired blind hole for installing the implant, while avoiding making accidental other holes or an undesirable oval osteotomy (hole) in the process of making the desired blind hole.

According to an embodiment, a drill member for making a blind hole in a bone under a soft tissue for installing an implant is disclosed. The drill member includes a stepped drill bit that includes a conical section, an intermediate section and a proximal section. The drill bit includes a conical width of a conical profile including an extrapolated outer surface of the conical section, an intermediate width of an intermediate profile comprising an extrapolated outer surface of the intermediate section and a proximal width of a proximal profile comprising an extrapolated outer surface of the proximal section. The conical width and/ or intermediate width is smaller than the proximal width. The proximal profile comprising the proximal width is adapted to receive at least a part of a first section of a drill head of a second drill member.

The soft tissue includes the skin (including the epidermis and the dermis), the subcutaneous fat and muscle tissue like periost. The blind hole in the underlying bone tissue has a width and depth matching the dimensions of the implant so that the implant may be screwed into the hole. In an embodiment, the implant includes an implant of a bone anchored hearing aid.

In an embodiment, the proximal width is larger than a first width of the first section of the drill head of a second drill member. This allows for completely receiving the first width of the first section in the proximal width. In another embodiment, the proximal width is either same or marginally smaller than the first width of the first section. However, in this embodiment, the proximal width is wide enough to receive at least a distal end of the first section of the second drill member, thus receiving at least a part of the first section. Therefore, in both embodiments, the proximal width is adapted to receive at least a part of the first section.

Providing the proximal width that is adapted to receive at least a part of the first section is particularly useful because the second drill member may be positioned precisely in a preliminary hole generated by using the drill member and challenges relating to finding the preliminary hole for making the blind hole or accidentally making a new hole using the second drill member is avoided. Furthermore, placing at least a part of the first section in the preliminary hole provides a tactile feedback, thus confirming correct placement of the second drill member for generation of the blind hole. This is especially useful while working in a visually constrained working space of bone drilling.

Therefore, according to an embodiment, the proximal width is adapted to receive at least a part of the first section of the drill head of a second drill member such that receiving of the at least a part of the first section in the proximal width provides a tactile feedback confirming positioning of the first section in the preliminary hole.

In general, the stepped drill bit is defined such that the drill bit includes at least two sections such that the width of the sections is different. The at least two sections may usually include three or more sections. Typically, the width would be such that the width of the section that is closer to the bone to be drilled is smaller than the width of the section that is farther away from the bone to be drilled. For each section, in different embodiments, the width may be constant, varying or a combination thereof.

The conical section is generally described as a drill bit section that is closest to the bone during drilling. The conical section usually include a pointed end interfacing with the bone. Although the term "pointed" is used but the term includes any known structure, interfacing that bone at the start of drilling, that facilitates piercing of the bone for creation of the preliminary hole. Typically, length of the conical section is less than those of the intermediate section and/ or the proximal section.

The intermediate section and the proximal section may be separately defined sections of a step-profile such that the width of the proximal section is more than that of the intermediate section. However, in another embodiment, a single section may include both the intermediate section and the proximal section. In this scenario, a part of the single section closer to the bone while drilling will be considered as the intermediate section and other part, relatively away from the bone, of the single section is considered as the proximal section. In different implementations of this embodiment, the width along the intermediate length and the proximal length may vary continuously such that the width at the beginning of the intermediate length, i.e. at an interface of the conical section and intermediate section, is lesser than the width at the end of the proximal section, i.e. width that is adapted to receive at least a part of the first section of the drill head of the second drill member.

In an embodiment, generating the blind hole is proposed as a multi-stage process wherein the preliminary hole using the drill member is made. This is followed by placing at least a part of the first section of the drill head of the second drill member in the proximal profile, including the proximal width, of the preliminary hole. It is apparent that two stage process and devices for creating the blind hole is disclosed. However, in other embodiments, a multi-stage process using the disclosed principle may also be used and such multi-stage process is within scope of this disclosure. For example, using a first drill member, a first preliminary hole of a specific dimension is created. This is followed by positioning a conical section of a second drill member in a first proximal width of the first preliminary hole for creating a second preliminary hole by modifying the dimensions of the first preliminary hole. Later, a first "second drill member" is received in the proximal width of the second preliminary hole to create a blind hole. Additionally, if required, a second "second drill member" may also be received in the blind hole and another blind hole in the bone may be generated by changing the dimensions of the blind hole generated by the first "second drill member", the another blind hole is used to install the implant in the bone. The two or multi-staged drilling method provides a more controlled way of generating the blind hole by overcoming the complexity of creating a blind hole of desired dimension in a single attempt.

For the drill member, section-specific profile includes an extrapolated outer surface that represents inner surface of the preliminary hole that is created by rotation of the specific section of the stepped drill bit. In other words, the extrapolated outer surface of a drill bit section is same as the inner surface of the preliminary hole created by the drill bit section in the bone.

The stepped drill bit includes the conical width of the conical section, intermediate width of the intermediate section and proximal width of the proximal section. In addition, the stepped drill bit includes a conical length of the conical width, an intermediate length of the intermediate width and a proximal length of the proximal width. Using the stepped drill bit defines the preliminary hole in the bone tissue. The preliminary hole includes a preliminary length comprising a conical length, intermediate length and at least a part of a proximal length and a preliminary width that includes the conical width, intermediate width and proximal width. In another embodiment, the preliminary length may include the proximal length instead of a part of a proximal length.

In an embodiment, the conical length is substantially smaller than the intermediate length and/ or proximal length. In different embodiments, the conical length is less than about 0.6 times, or lower than the intermediate length such as less than about 0.55 times, 0.5 times, 0.45 times, 0.40 times, 0.35 times, 0.25 times, 0.2 times, 0.15 times , 0.1 times and so on. Having a substantially smaller conical length relative to the intermediate length provides higher stability during drilling compared to the drill having only a conical drill bit or a substantially long conical section. In other different embodiments, the conical length is less than about 0.6 times, or lower than the proximal length such as less than about 0.55 times, 0.5 times, 0.45 times, 0.40 times, 0.35 times, 0.25 times, 0.2 times, 0.15 times , 0.1 times and so on. Having a substantially smaller conical length relative to the proximal length provides higher stability during drilling compared to the drill having only a conical drill bit or a substantially long conical section.

In an embodiment, the proximal width is at least about 1.3 times or more than the conical width. For example, the proximal width is at least about 1.4 times or 1.5 times or 1.6 times or 1.7 times or 1.8 times or 1.9 times or 2 times and so on. Having a substantially larger proximal width relative to the conical width provides higher stability during drilling and also to creates the preliminary hole that is adapted to receive the first width of the second drill compared to the drill having only a conical drill bit or a narrow conical section. In an embodiment, the intermediate width is at least about 1.2 times or more than the conical width. For example, the proximal width is at least about 1.4 times or 1.5 times or 1.6 times or 1.7 times or 1.8 times or 1.9 times or 2 times and so on. Using the proposed stepped drill bit where the proximal width is larger than the conical width and/ or intermediate width also provides strength to the drill bit, thus avoiding breaking of the drill bit.

In an embodiment, the drill head of the second drill member includes the first section and a second section such that the first width of the first section is lesser than a second width of the second section. The first section and the second section together may define the drill head of the second drill member. At least a part of the first section is adapted to accommodate in the proximal profile. When at least a part of the first section is received in the proximal width, the first section is closer to the bone relative to distance of the second section from the bone to be drilled. It is apparent that the second drill member may include more sections such as 3 sections, 4 sections and so on where at least a part of the first section is adapted to accommodate in the proximal profile. In an embodiment, the sections farther away from the bone include width that is larger than the width of the section that are relatively closer to the bone.

In an embodiment, the second drill member includes a stepped drill head. Although the illustrative figures (included later) show two step drill head. However, it is apparent and within scope of the disclosure that more than two steps may be included in the drill head. The first width and/ or the second width may include a constant width and/ or varying width along the length of the first section and the second section.

In an embodiment, where the first width of the first section is constant and less than that of the proximal width, the first section is adapted not to widen the proximal width during generation of the blind hole.

In an embodiment, where the first width varies along a first length of the first section, a first section part closer to the bone includes a width lesser than the width of first section part that is away from the bone. At least a part of the first section part that is closer to the bone is adapted to be received in the proximal width of the preliminary hole. In this case, the varying width of the first section may also be adapted to widen the proximal width provided the width at the interface of the first section and the second section and/ or along the length of the first section is wider than the proximal width. In another embodiment, the drill head of the second drill member includes a conical drill head with at least a part of the drill head, i.e. a conical part closer to the bone during drilling, being adapted to be received in the proximal width. A conical part closer to the bone includes a width that is smaller than that of a conical part that is away from the bone. This allows the conical drill head to widen the proximal width of the preliminary hole provided that the width along the length of the conical drill head increases to be more than the proximal width. If not, then the conical drill head will not widen the proximal width.

Using a drill head of the disclosed configuration where the width of the first section varies along the first length or a conical drill head allows for fine tuning the generation of the blind hole. Also, because the implant may be conical or stepped shaped, therefore using the drill head of a conical or stepped configuration is useful in creating a blind hole that matches with the profile of the implant, i.e. a conical shaped implant or a stepped shaped implant respectively.

The disclosure is described by way of a stepped second drill member. However, in an embodiment, the disclosure is applicable for a second drill member comprising a conical drill that includes a conical drillhead as well. In this situation, the first section and the second section may be considered to be part of a conical drill head such that the width of the conical drill head varies along the combined length of the first section and the second section such that a first width of the conical part closer to the bone while drilling is smaller than that of a second width of the conical part that is away from the bone while drilling. In such a situation, the phrase "the proximal profile comprising the proximal width is adapted to receive at least a part of a first section of a drill head of a second drill member" is to be interpreted to include "the proximal profile comprising the proximal width is adapted to receive at least a part of the conical drill head that is closer to the bone while drilling". Similarly, the phrase "at least a part of the first section is adapted to accommodate in the proximal width" is to be interpreted to include "at least a part of the conical part of the conical drill that is closer to the bone while drilling is adapted to accommodate in the proximal width". Similar interpretation may be applicable for the first section to include a part of the conical drill head closer to the bone while drilling and second section to include a part of the conical drill head that is away from the bone while drilling. In another embodiment, for example in the disclosure of the kit, the at least one second drill member includes at least one conical drill. In yet another embodiment, for example in disclosure of the method, receiving of at least a part of the first section include receiving at least a part of the conical part of the conical drill head that is closer to the bone while drilling.

In one embodiment, when at least a part of the first section of the second drill member is received in the proximal profile, the first section is adapted to widen the conical width and intermediate width of the preliminary hole with or without widening the proximal width during drilling using the second drill member. Additionally or alternatively, when at least a part of the first section is received in the proximal profile, the second section is adapted to widen the conical width and intermediate width of the preliminary hole with or without widening the proximal width during drilling using the second drill member. The drilling along a drilling depth using the second drill member defines the blind hole, comprising a blind depth, that is adapted to receive the implant for installation. The blind depth includes the length of the blind hole that is made in the bone at the end of the staged drilling process. Typically, the blind depth is pre-determined in accordance with the length of the implant. For example, the blind length for a bone anchored hearing aid would depend upon fixture length that is to be installed in the bone.

In different embodiments, the conical width and/ or intermediate width and/ or proximal width is selected from a group of constant width, increasingly varying width and a combination of a constant width and a varying width. For example, the intermediate width is selected from a constant width along the intermediate length or increasingly varying width from a conical interface to a proximal interface and a combination of a constant width and a varying width along the intermediate length.

In different embodiments, the drill member includes at least two flutes such as two or three or four or more along a length of the stepped drill bit. Similarly, the second drill member may also include at least two flutes such as three or four or more flutes along a length of the drill head.

In different embodiments, the drill member includes flutes that are selected from group of straight flutes, helical flutes, regular flutes, irregular flutes and a combination thereof. Similarly, the second drill member includes flutes that are selected from group of straight flutes, helical flutes, regular flutes and irregular flutes. The straight flutes may be circularly straight, longitudinally straight and a combination thereof. The regular flute may refer to flutes that are continuous along the circular and/ or longitudinal periphery of the drill bit/ drill head. The irregular flute may refer to flutes that are discontinuous along the circular and/ or longitudinal periphery of the drill bit/ drill head.

It is generally preferred that the drill member and the second drill member include large flutes. Hereby, this ensures that the bone tissue can be sufficiently cooled down and also bone fragments generated during the drilling may be flushed away from the hole.

In one embodiment, the implant is an implant module, such as the fixture, of a bone anchored hearing aid.

In an embodiment, a drill kit for making a blind hole in a bone under a soft tissue for installing the implant is disclosed. The kit includes at least one stepped drill member and at least one second drill member. Each stepped drill member includes a stepped drill bit that includes a conical section, an intermediate section and a proximal section. The conical width of a conical profile includes an extrapolated outer surface of the conical section. The intermediate width of an intermediate profile includes an extrapolated outer surface of the intermediate section. The proximal width of a proximal profile incldues an extrapolated outer surface of the proximal section. The conical width and/ or the intermediate width are adapted such that the conical width and/ or intermediate width is smaller than the proximal width. Each second drill member includes a drill head that includes a first section. The first section includes a first width such that at least a part of a first section of a drill head of one of the at least one second drill member is adapted to be received in the proximal width of one of the at least one stepped drill member.

In an embodiment, using one of the at least one stepped drill member comprising the conical width, intermediate width and proximal width define a preliminary hole in a bone tissue, the preliminary hole comprising a preliminary length comprising a conical length, intermediate length and at least a part of a proximal length and a preliminary width comprising the conical width, intermediate width and proximal width.

In yet another embodiment, at least a part of the first section of one of the at least one second drill member is received in the proximal width of one of the at least one stepped drill member, the first section is adapted to widen the conical width and intermediate width of the one of the at least one stepped drill member in the preliminary hole with or without widening the proximal width. Additionally or alternatively, a second section of one of the at least one second drill member is adapted to widen the conical width and intermediate width of the one of the at least one stepped drill member in the preliminary hole with or without widening the proximal width, the drilling along a drilling depth defining the blind hole, comprising a blind depth, that is adapted to receive the implant for installation.

Instead of providing at least one drill member and at least one second drill member, in another embodiment, the kit includes a plurality of drill members and a plurality of second drill members.

Inclusion of the at least one drill member and at least one second drill member or the plurality of the drill members and plurality of second drill members allow for performing multi-stage drill process. This allows for generating different blind holes with different dimensions, thus providing flexibility in blind hole generation.

The kit disclosed hereinabove may include any of the features disclosed in the description that relates to the drill member and second drill member.

In yet another embodiment, a method for making a blind hole in a bone under a soft tissue for installing an implant is disclosed. The method includes inserting a stepped drill member into an incision hole available in a soft tissue. A preliminary hole is generated in a bone tissue, using the inserted drill member that includes a stepped drill bit. The preliminary hole includes a conical width of a conical profile including an extrapolated outer surface of a conical section of the stepped drill bit, an intermediate width of an intermediate profile including an extrapolated outer surface of the intermediate section of the stepped drill bit and a proximal width of a proximal profile including an extrapolated outer surface of the proximal section of the stepped drill bit. The conical width and/ or intermediate width is smaller than the proximal width. The inserted drill member is then removed. Thereafter, at least a part of the first section of a second drill member is positioned within the proximal profile, i.e. at least a part of the first section is received in the proximal width. Using the second drill member, the bone is drilled to make a blind hole in a bone tissue by widening the preliminary hole; and finally the implant is installed in the blind hole.

In an embodiment, after receiving a part of the first section of the second drill member in the proximal profile, widening of the preliminary hole includes widening the conical width and intermediate width with or without widening the proximal width using the first section of the second drill member. Additionally or alternatively, after receiving a part of the first section in the proximal profile, widening the preliminary hole includes widening the conical width and intermediate width of the preliminary hole with or without widening the proximal width using the second section of the second drill member. The drilling along a drilling depth using the second drill member defines depth of the blind hole that is adapted to receive the implant for installation.

In an embodiment, prior to inserting the stepped drill member an incision hole is provided in a soft tissue by pressuring a sharp blade of a cylindrical hollow punch member through the soft tissue. This followed by pulling out the cylindrical hollow punch member; and removing the punched out soft tissue from the incision hole.

The method may be implemented by the drill member and/ or second drill member including at least one feature that are disclosed in the disclosure. The method may be implemented by the at least one drill member and at least one second drill member that are included in the kit.

In an embodiment, generating the blind hole is proposed as a multi-stage process wherein the preliminary hole using the drill member is made. This is followed by placing at least a part of the first section of the drill head of the second drill member in the proximal profile, including the proximal width, of the preliminary hole. It is apparent that two stage process and devices for creating the blind hole is disclosed. However, in other embodiments, a multi-stage process using the disclosed principle may also be used and such multi-stage process is within scope of this disclosure. For example, using a first drill member, a first preliminary hole of a specific dimension is created. This is followed by positioning a conical section of a second drill member in a first proximal width of the first preliminary hole for creating a second preliminary hole by modifying the dimensions of the first preliminary hole. Later, a first second drill member is received in the proximal width of the second preliminary hole to create a blind hole. Additionally, if required, a second second drill member may also be received in the blind hole and another blind hole in the bone may be generated by changing the dimensions of the blind hole generated by the first second drill member, the another blind hole is used to install the implant in the bone.

The disclosure also incorporates the description included in currently pending application EP13197428.9, EP14190289.0 and US 14/570893, which are incorporated here by reference.

According to an embodiment, a drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member and/ or second drill member for drilling the preliminary hole and/ or blind hole in the bone respectively. The drill guide further includes a flange at a proximal end section of the drill guide. The flange extends perpendicular or substantially perpendicular to the longitudinal axis of the cylindrical portion. The drill guide has a length that is equal or greater than thickness of the soft tissue. A distal end of the drill guide, when inserted in a hole, is adapted to rest against a bone surface that interfaces with the soft tissue.

In different embodiments, the flange may include different geometric shapes such as a circular, elliptical, rectangular, etc. Furthermore, the flange may include at least one grip at a periphery of the flange. A grip surface facing outward may include grip enhancers. The grips allow for not only handling the drill guide but also to push the drill guide in an incision hole or for making the incision hole if the drill guide includes blade(s).

In an embodiment, an outer cylindrical wall of the drill guide abuts the soft tissue when inserted and an inner cylindrical wall guides the drill member and/ or second drill member for generating the preliminary hole and/ or blind hole.

In an embodiment, a dual drill guide system is used. The dual drill guide system includes an outer drill guide and an inner drill guide. In case wall member of the outer drill guide bends radially inward, such as because of the pressure applied by soft tissue surrounding the wall member, an inner drill guide may be inserted in the canal of the outer drill guide. This allows the wall members of the outer drill guide to be pushed radially outwards.

In an embodiment, the drill guide includes at least one cooling channel including at least one proximal channel port positioned at the proximal end section and at least one distal channel port positioned at a distal end section of the drill guide. The at least one proximal channel port is adapted to receive a cooling fluid. The at least one cooling channel is adapted to transmit the cooling fluid from the at least one proximal channel port to the at least one distal channel port, which is adapted to expel the cooling fluid out of the at least one cooling channel. The at least one distal channel port is in fluid connection with respective at least one channel. Thus, the cooling channels of the drill guide is adapted to provide adequate irrigation of the bone tissue during a drilling procedure. The skilled person may choose cooling fluid that suits their specific requirement such as an NaCl solution.

In another embodiment, the distal end section of the drill guide includes a sharp blade at the distal end of the drill guide. Additionally or alternatively, the drill guide may further include spatially separated longitudinal and/ or circular blades along length of the distal end section. The blade and or blades may be selected from a group consisting of regular edge, serrated edge and a combination thereof. The serrated edges at the distal end, resting against a bone surface interfacing the soft tissue, of the guide might be useful in order to provide stability to the guide when the guide is in position because the serrated edges at the distal end are adapted to grip, when pressed, the surface of the bone.

During the drilling of the blind hole, bone dust/ debris is generated. The bone dust or debris rises up along the flutes of the drill member and/ or second drill member. In order to allow such debris to be removed more efficiently, according to an embodiment, the drill guide includes at least one opening at the proximal end section of the drill guide. During the drilling process, the dust/ bone debris rises along flutes of the drill member and the at least one opening is adapted to allow the risen dust/ bone debris to be expelled from the at least one opening during the drilling process. The at least one opening is positioned along the length of the drill guide such that the at least opening is not surrounded by the soft tissue when the drill guide is positioned in the hole. Such opening may include different shapes such as a slit or hole across the thickness of the walls of the drill guide.

In yet another embodiment, an upper surface of the flange of the drill guide includes a plurality of spatially separated ribs. Each rib of the plurality of ribs includes a first end in immediate proximity to the drill entry port and a second end at a predetermined distance from the drill entry port of the drill guide. The drill entry port is adapted to receive the drill member and/ or second drill member. Neighboring ribs of the plurality of ribs form a bounded area between the neighboring ribs. The bounded area is adapted to collect the bone dust/ debris that is exported upward from the bone drilling site along the flutes of the drill member and expelled from the drill entry port.

According to an embodiment, the drill member and/ or the second drill member includes a drill stop member. The stop member may be adapted to abut and rest against the upper surface of the flange or against a plurality of spatially separated ribs of the drill guide. The possibility of having the stop member resting against the upper surface of the flange surface or against the plurality of spatially separated ribs ensures that correct and reproducible depth of the preliminary hole and/ or blind hole depth.

According to yet another embodiment, a healing cap adapted to be used post implant is disclosed. The healing cap is designed such that the processing unit such as a sound processor of a bone anchored hearing aid can be fitted to the abutment immediately after the surgery. Thus, time delay with conventional solutions where sound processor is attached only after healing post-surgery has occurred can be avoided. According to another embodiment, the healing cap includes a plurality of evenly or unevenly spaced ridges at the lower surface of the healing cap. Each ridge of the plurality of ridges include a distal end that is adapted to abut skin. A plurality of distal ends of the plurality of ridges create pockets of lower surface sections that are at a distance from the skin.

In an embodiment, a graded probe adapted to measure depth of the hole in the soft tissue is provided. The graded probe includes a plurality of markings representing distance in mm from a distal end of the probe, the distal end being adapted to rest against the bone surface that interfaces with the soft tissue. The markings represents distances such as between 8 mm - 16 mm such as 9 mm, 9.5 mm, 12 mm, 13 mm, 14 mm, 16mm etc.. Other finer markings are also possible and within the scope of this disclosure.

The kit described previously in the disclosure may also include additional components such as at least one of a punch, a drill guide, a healing cap, a wrap member, abutment, and an implant. The kit may also include a graded probe.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of the other aspects. These and other aspects, features and/or technical effect will be apparent from and elucidated with reference to the illustrations described hereinafter in which:
Fig. 1A illustrates a drill member according to an embodiment of the disclosure;
Fig. 1B illustrates a drill member according to an embodiment of the disclosure;
Fig. 1C illustrates a drill member according to an embodiment of the disclosure;
Fig. 1D illustrates a drill member according to an embodiment of the disclosure;
Fig. 2 illustrates a second drill member including helical flutes according to an embodiment of the disclosure;
Fig. 3 illustrates straight flutes of a second drill member according to an embodiment of the disclosure;
Fig. 4A illustrates soft tissue and bone where the implant is to be installed according to an embodiment of the disclosure;
Fig. 4B illustrates the soft tissue with an incision hole according to an embodiment of the disclosure;
Fig. 5 illustrates profiles in the bone of a preliminary hole generated by the drill member according to an embodiment of the disclosure;
Fig. 6A illustrates the preliminary hole generated by the drill member according to an embodiment of the disclosure;
Fig. 6B illustrates the first section of the second drill member received in the proximal width according to an embodiment of the disclosure;
Fig. 6C illustrates a blind hole generated by the second drill member according to an embodiment of the disclosure;
Fig. 7A illustrates the preliminary hole generated by the drill member according to another embodiment of the disclosure;
Fig. 7B illustrates the first section of the second drill member received in the proximal width according to another embodiment of the disclosure;
Fig. 7C illustrates a blind hole generated by the second drill member according to another embodiment of the disclosure;
Fig. 8A illustrates a schematic view of an implant screw arranged above the incision hole and blind hole generated according to an embodiment of the disclosure;
Fig. 8B illustrates a schematic cross-sectional view of the implant screw attached to the bone according to an embodiment of the disclosure; and

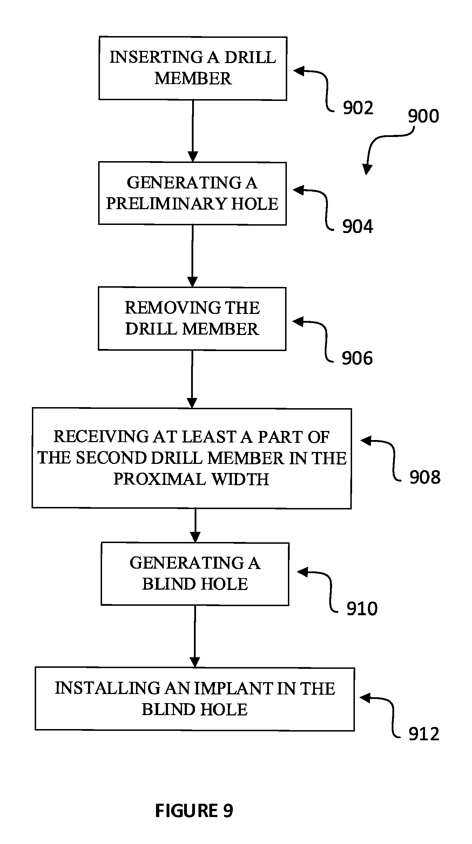

Fig. 9 illustrates a method for generating a blind hole according to an embodiment of the disclosure.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details.

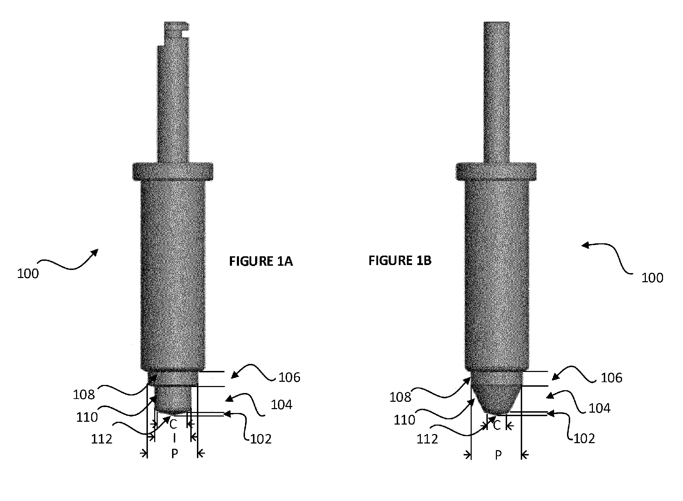

Fig. 1A-Fig. 1D illustrate drill members 100 according to different embodiments of the disclosure. The figures show stepped drill member bit comprising a conical section 112, an intermediate section 110 and a proximal section 108. The conical section includes a conical width C of a conical length 102. The intermediate section includes an intermediate width I of an intermediate length 104. The proximal section includes a proximal width P of proximal length 106.

As shown, the conical section usually includes a pointed end that interfaces with the bone. The term "pointed" includes any known structure that facilitates piercing of the bone for creation of the preliminary hole.

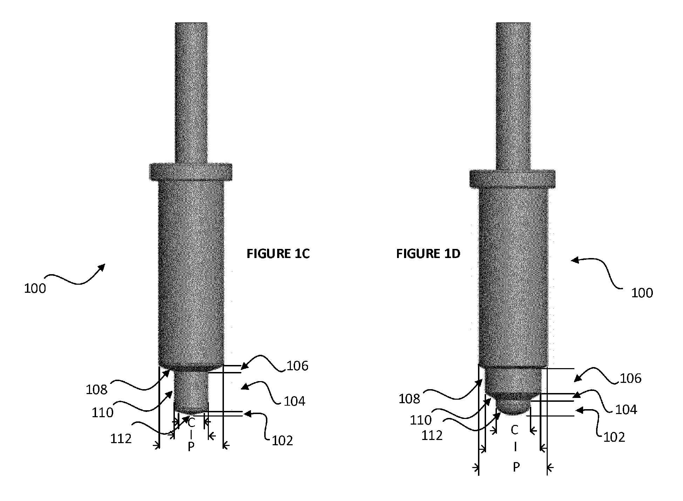

In some embodiments, the width for one or more section is constant along the respective length of the one or more section (For example, 110 in Figs. 1A, 1C; and 108 in Figs. 1A, 1B, 1D). In other embodiments, the width of one or more section is varying along the respective length of the one or more section (For example, 112 in Figs. 1A-1D, 110 in Figs. 1B, Figs. 1D; and 106 in Fig. 1C).

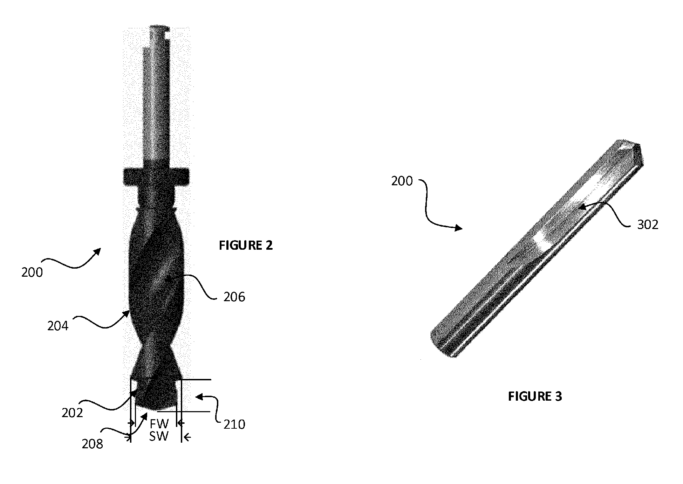

Fig. 2 illustrates a second drill member including helical flutes according to an embodiment of the disclosure. The second drill member 200 includes a first section 202 and a second section 204, the first section and the second section together defining the drill head. The first section includes a distal end 208. The first end also includes a first width FW of a first length 210. The second section includes a second width SW. The second drill member 200 may further include flutes 206. Figure 2 illustrates helical flutes.

Fig. 3 illustrates straight flutes 302 of a second drill member 200 according to an embodiment of the disclosure. The second drill member 200 includes the features 202, 204, 208, 210, FW and SW of the second drill illustrated in Fig. 2.

In different embodiments, the drill member 100 and/ or the second drill member 200 is configured to include at least two flutes such as two or three or four or more along a length of the stepped drill bit and/ or along a length of the drill head respectively. In different embodiments, the flutes are selected from group of straight flutes, helical flutes, regular flutes, irregular flutes and a combination thereof.

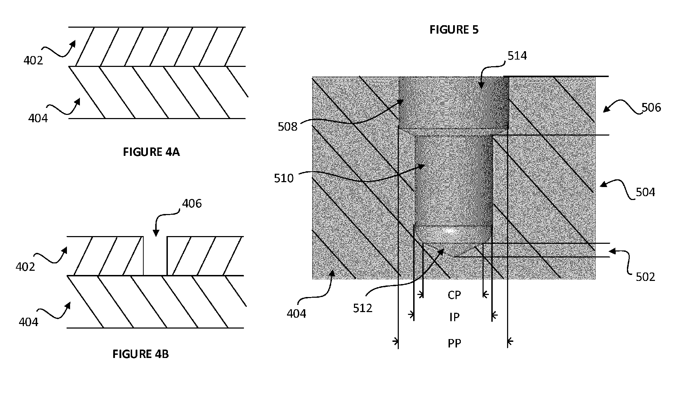

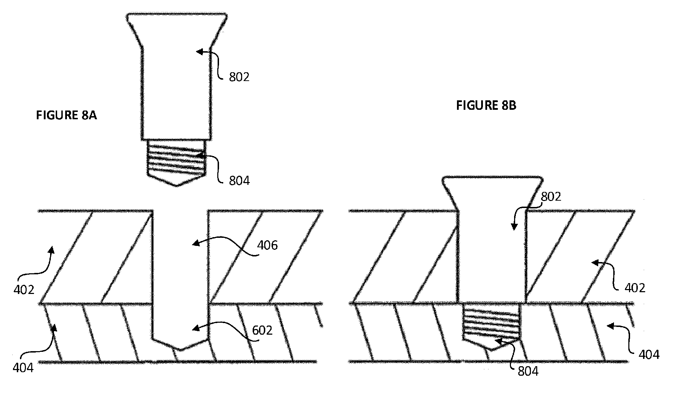

Fig. 4A illustrates a soft tissue 402 and bone 404 where an implant (802, Fig. 8) is to be installed according to an embodiment of the disclosure. In an embodiment, where an implant includes an implant (like fixture) of a bone anchored hearing aid, the bone would include the skull bone of the recipient of the implant.

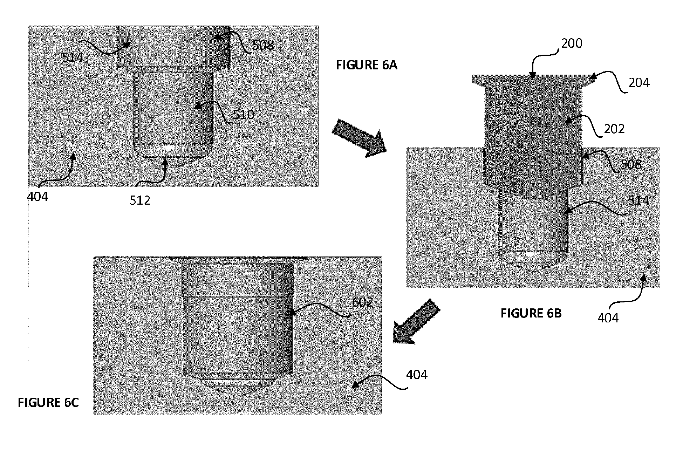

Fig. 4B illustrates the soft tissue 402 with an incision hole 406 in the soft tissue according to an embodiment of the disclosure. The incision hole 406 is usually made using a hollow punch (typically cylindrical) having sharp blades and/ or a drill guide comprising blades. The punched soft tissue may be picked out with tweezers or another suitable tool. When the punching of the incision hole 406 has been carried out, the bone tissue 404 is made available for creation of the preliminary hole (514, Fig. 5) and later a blind hole (602, Figs. 6C, 7C, 8A), which is used for installing the implant.

Fig. 5 illustrates profiles in the bone of a preliminary hole generated by the drill member according to an embodiment of the disclosure. The profiles are created in the bone 404 by using the drill member (100, Fig. 1) comprising the stepped drill bit. It is apparent that the profiles of the preliminary hole 514, a cavity generated in the bone tissue, correspond to the stepped structure of the drill bit of the drill member. The figure shows section-specific profiles that includes extrapolated outer surface of the stepped drill bit, thus representing inner surface of the preliminary hole 514 that is created by rotation of the specific section of the stepped drill bit. In other words, the extrapolated outer surface of a drill bit section is same as the inner surface of the preliminary hole created by the drill bit section in the bone. The preliminary hole 514 in the bone 404 includes a conical profile 512, an intermediate profile 510, a proximal profile 508. The conical profile includes a conical width CP and a conical length 502. The intermediate profile includes an intermediate width IP of an intermediate length 504. The proximal profile includes a proximal width PP of a proximal length 506. It is apparent that the inner surface of the preliminary hole 514 corresponds to the stepped structure of the stepped drill bit. In particular, 502, 504, 506, 508, 510, 512, CP, IP, and PP of the preliminary hole 514 corresponds to 102, 104, 106, 108, 110, 112, C, I, and P (refer Fig. 1) of the drill member respectively.

Fig. 6A illustrates the preliminary hole 514 generated by the drill member 100 in the bone tissue 404 according to an embodiment of the disclosure. The preliminary hole includes the conical profile 512, the intermediate profile 510 and the proximal profile 508. As a next step, after removal of the drill member 100 from the preliminary hole 514, at least a part of the second drill member 200 is received in the preliminary hole. In a particular embodiment of Fig. 6B, the first section 202 of the second drill member 200 is received in the proximal width 508 of the preliminary hole 514. The second drill member is used to widen the preliminary hole for creating a blind hole 602, which in accordance with an embodiment is shown in Fig. 6C.

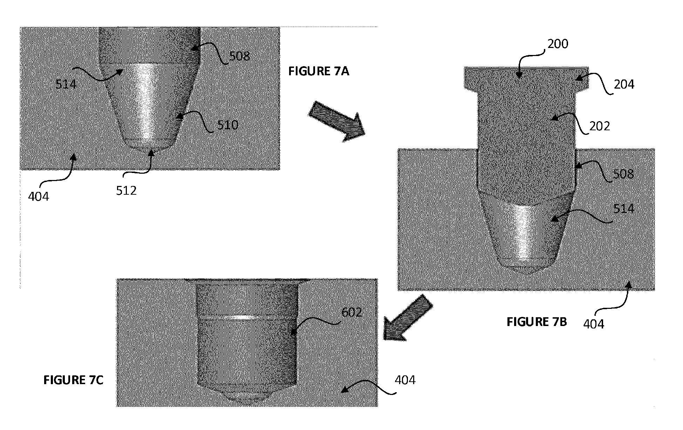

Fig. 7A illustrates the preliminary hole 514 generated by the drill member 100 in the bone tissue 404 according to an embodiment of the disclosure. The preliminary hole includes the conical profile 512, the intermediate profile 510 and the proximal profile 508. As a next step, after removal of the drill member 100 from the preliminary hole 514, at least a part of the second drill member 200 is received in the preliminary hole. In a particular embodiment of Fig. 7B, the first section 202 of the second drill member 200 is received in the proximal width 508 of the preliminary hole 514. The second drill member is used to widen the preliminary hole for creating a blind hole 602, which in accordance with an embodiment is shown in Fig. 7C. This embodiment differs from the embodiment of Fig. 6 in that the intermediate profile includes an intermediate width that varies along the intermediate length. This embodiment corresponds to the stepped drill bit of Fig. 1B whereas Fig. 6 uses the drill member 100 of Fig. 1A.

Fig. 8A illustrates a schematic view of an implant screw 802 arranged above the incision hole 406 made in the soft tissue 402 and blind hole 602 generated in the bone tissue 404 according to an embodiment of the disclosure. The implant screw 802 may include a threaded section 804 adapted for screwing into the blind hole. Fig. 8B illustrates a schematic cross-sectional view of the implant screw 802 attached to the bone 404 according to an embodiment of the disclosure. In one embodiment, the implant screw is a fixture of the bone anchored hearing aid.

According to an embodiment, a method 900 for creating a blind hole is disclosed. At step 902, inserting a stepped drill member into an incision hole available in a soft tissue. At 904, a preliminary hole is generated in a bone tissue, using the inserted drill member that includes a stepped drill bit. The preliminary hole includes a conical width of a conical profile including an extrapolated outer surface of a conical section of the stepped drill bit, an intermediate width of an intermediate profile including an extrapolated outer surface of the intermediate section of the stepped drill bit and a proximal width of a proximal profile including an extrapolated outer surface of the proximal section of the stepped drill bit. The conical width and/ or intermediate width is smaller than the proximal width. At 906, the inserted drill member is then removed. At 908, at least a part of the first section of a second drill member is positioned within the proximal profile, i.e. at least a part of the first section is received in the proximal width. At 910, using the second drill member, the bone is drilled to make a blind hole in a bone tissue by widening the preliminary hole. Finally, at 912, the implant is installed in the blind hole.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

Accordingly, the scope should be judged in terms of the claims that follow.

## Claims

1. A drill member for making a blind hole in a bone under a soft tissue for installing an implant, the drill member comprising a stepped drill bit comprising a conical section, an intermediate section and a proximal section such that
a conical width of a conical profile comprising an extrapolated outer surface of the conical section and/ or an intermediate width of an intermediate profile comprising an extrapolated outer surface of the intermediate section is smaller than a proximal width of a proximal profile comprising an extrapolated outer surface of the proximal section, and
the proximal profile comprising the proximal width is adapted to receive at least a part of a first section of a drill head of a second drill member.

2. The drill member according to claim 1, wherein using the stepped drill bit comprising the conical width, intermediate width and proximal width defines a preliminary hole in a bone tissue, the preliminary hole comprising a preliminary length comprising a conical length, intermediate length and at least a part of a proximal length and a preliminary width comprising the conical width, intermediate width and proximal width.

3. The drill member according to any of the preceding claims, wherein
the drill head comprises the first section and a second section such that the first width of the first section is lesser than a second width of the second section; and
at least a part of the first section is adapted to accommodate in the proximal width.

4. The drill member according to any of the preceding claims, wherein when at least a part of the distal end is received in the proximal profile, the first section is adapted to widen the conical width and intermediate width of the preliminary hole with or without widening the proximal width and/ or the second section is adapted to widen the conical width and intermediate width of the preliminary hole with or without widening the proximal width, the drilling along a drilling depth defining the blind hole, comprising a blind depth, that is adapted to receive the implant for installation.

5. The drill member according to any of the preceding claims, wherein the conical width and/ or intermediate width and/ or proximal width is selected from a group of constant width, increasingly varying width and a combination of a constant width and a varying width.

6. The drill member according to any of the preceding claims, wherein the drill member and/ or the second drill member comprises at least two flutes.

7. The drill member according to any of the preceding claims, wherein the drill member and/ or the second drill member comprises flutes that are selected from a group consisting of a straight flutes, helical flutes, regular flutes, irregular flutes and a combination thereof.

8. The drill member according to any of the preceding claims, wherein the implant is an implant module of a bone anchored hearing aid.

9. A drill kit for making a blind hole in a bone under a soft tissue for installing the implant, the kit comprising at least one stepped drill member and at least one second drill member, wherein
each stepped drill member comprises a stepped drill bit comprising a conical section, an intermediate section and a proximal section such that a conical width of a conical profile comprising an extrapolated outer surface of the conical section and/ or an intermediate width of an intermediate profile comprising an extrapolated outer surface of the intermediate section is smaller than a proximal width of a proximal profile comprising an extrapolated outer surface of the proximal section, and
each second drill member comprises a drill head comprising a first section comprising a first width such that at least a part of a first section of a drill head of one of the at least one second drill member is adapted to be received in the proximal width of one of the at least one stepped drill member.

10. The kit according to any of the claims 9, wherein using one of the at least one stepped drill member comprising the conical width, intermediate width and proximal width define a preliminary hole in a bone tissue, the preliminary hole comprising a preliminary length comprising a conical length, intermediate length and at least a part of a proximal length and a preliminary width comprising the conical width, intermediate width and proximal width.

11. The kit according to any of the claims 9-10, wherein when at least a part of the first section of one of the at least one second drill member is received in the proximal width of one of the at least one stepped drill member, the first section is adapted to widen the conical width and intermediate width of the one of the at least one stepped drill member in the preliminary hole with or without widening the proximal width and/ or a second section of one of the at least one second drill member is adapted to widen the conical width and intermediate width of the one of the at least one stepped drill member in the preliminary hole with or without widening the proximal width, the drilling along a drilling depth defining the blind hole, comprising a blind depth, that is adapted to receive the implant for installation.

12. The kit according to any of the claims 9-11, further comprising any of the features included in claims 1-7.

13. A method for making a blind hole in a bone under a soft tissue for installing an implant, the method comprising
inserting a stepped drill member into an incision hole available in a soft tissue;
drilling a preliminary hole in a bone tissue, using the inserted drill member comprising a stepped drill bit such that the preliminary hole comprises a conical width of a conical profile comprising an extrapolated outer surface of a conical section of the stepped drill bit, an intermediate width of an intermediate profile comprising an extrapolated outer surface of the intermediate section of the stepped drill bit and a proximal width of a proximal profile comprising an extrapolated outer surface of the proximal section of the stepped drill bit, the conical width and/ or intermediate width being smaller than the proximal width;
removing the inserted drill member;
positioning at least a part of a first section of a second drill member within the proximal profile;
drilling, using the second drill member, to make a blind hole in a bone tissue by widening the preliminary hole; and
anchoring the implant in the blind hole.

14. The method according to claim 13, wherein prior to inserting the stepped drill member
providing an incision hole in a soft tissue by pressuring a sharp blade of a cylindrical hollow punch member through the soft tissue;
pulling out the cylindrical hollow punch member; and
removing the punched out soft tissue from the incision hole.

15. The method according to any of the claims 13-14, wherein the drill member and the second drill member comprises features of any of the claims 1-7.
